(19) Europäisches Patentamt
European Patent Office
Office européen des brevets

(11) **EP 2 335 704 A1**

(12) **DEMANDE DE BREVET EUROPEEN**

(43) Date de publication:
**22.06.2011 Bulletin 2011/25**

(21) Numéro de dépôt: **10290628.6**

(22) Date de dépôt: **26.11.2010**

(51) Int Cl.:
*A61K 31/593* (2006.01)      *A61K 33/24* (2006.01)
*A61K 47/40* (2006.01)       *A61K 45/06* (2006.01)
*A61P 19/00* (2006.01)

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(30) Priorité: **27.11.2009 FR 0905706**

(71) Demandeur: **Les Laboratoires Servier
92284 Suresnes Cedex (FR)**

(72) Inventeurs:
• **Briault, Gilles
45480 Saint Peravy Epreux (FR)**

• **Quenault, Xavier
45130 Baule (FR)**
• **Poirier, Cécile
45000 Orleans (FR)**
• **Pean, Jean-Manuel
45000 Orleans (FR)**

(74) Mandataire: **Bestel, Delphine
Les Laboratoires Servier
Direction Brevets
35 rue de Verdun
92284 Suresnes Cedex (FR)**

(54) **Composition pharmaceutique comprenant un sel de strontium, de la vitamine D et une cyclodextrine**

(57) Composition pharmaceutique comprenant un sel de strontium, de la vitamine D et une cyclodextrine.

EP 2 335 704 A1

**Description**

La présente invention concerne une composition pharmaceutique comprenant un sel de strontium, de la vitamine. D et une cyclodextrine, ainsi que son utilisation dans le traitement des maladies osseuses et de l'arthrose.

L'utilisation en thérapeutique des sels de strontium a été décrite notamment dans les brevets EP 0 415 850, EP 0 813 869, EP 1 534 305 et EP 1 845 082.

Des compositions contenant un sel de strontium et de la vitamine D ont été décrites de façon générique dans la demande de brevet WO 2004/098618. Des compositions pharmaceutiques contenant du ranélate de strontium et de la vitamine D ont été décrites dans la demande de brevet CN 1823764.

Le Demandeur a trouvé que la complexation de la vitamine D à une cyclodextrine améliore à la fois la stabilité et l'uniformité de teneur de la vitamine D au sein de la composition.

Par vitamine D, on entend le cholécalciférol (vitamine $D_3$), l'ergocalciférol (vitamine $D_2$), le calcidiol (25-hydroxy-vitamine $D_3$,) ou le calcitriol (1,25-dihydroxy-vitamine $D_3$). La vitamine D préférentiellement utilisée dans les compositions selon l'invention est la vitamine $D_3$.

Parmi les cyclodextrines utilisables dans les compositions selon l'invention, on peut citer à titre non limitatif les a-cyclodextrines, les (β-cyclodextrines et les γ-cyclodextrines, substituées ou non.

Parmi les cyclodextrines substituées, on pourra citer plus particulièrement les a-cyclodextrines, β-cyclodextrines et γ-cyclodextrines substituées par un ou plusieurs groupements méthyle, hydroxypropyle ou sulfobutyléther.

Les cyclodextrines préférées sont les (β-cyclodextrines substituées.

Parmi les (β-cyclodextrines substituées, on pourra citer plus particulièrement les HPBCD

(hydroxypropyl-(β-cyclodextrines), les SBECD (sulfobutyléther-(β-cyclodextrines) et les (β-cyclodextrines méthylées ou partiellement méthylées telles que le DIMEB (heptakis(2,6-di-*0*-méthyl)-β-cyclodextrine), le RAMEB ((β-cyclodextrine statistiquement méthylée) ou le TRIMEB (heptakis (2,3,6-tri-*0*-méthyl)-β-cyclodextrine).

Parmi les sels de strontium, on pourra citer plus particulièrement le ranélate de strontium, le malonate de strontium, l'acétate de strontium, le L-ascorbate de strontium, l'aspartate de strontium, le borate de strontium, le camphorate de strontium, le carbonate de strontium, le cétoglutarate de strontium, le citrate de strontium, l'éthanesulfonate de strontium, le formate de strontium, le fumarate de strontium, le gluconate de strontium, le glutamate de strontium, l'hydrogénophosphate de strontium, le lactate de strontium, le L-lactate de strontium, le L-malate de strontium, le maléate de strontium, le méthanesulfonate de strontium, le nitrate de strontium, l'oxalate de strontium, le phosphate de strontium, le propanesulfonate de strontium, le succinate de strontium, le sulfate de strontium, le tartrate de strontium, ainsi que leurs hydrates.

Parmi les compositions pharmaceutiques selon l'invention, il sera cité plus particulièrement celles qui conviennent pour l'administration orale, et notamment les comprimés à avaler simples ou dragéifiés, les comprimés à croquer, les comprimés effervescents, les comprimés dispersibles, les comprimés sublinguaux, les gélules, et les granules pour sachets.

Outre le sel de strontium, la vitamine D et la cyclodextrine, les compositions pharmaceutiques selon l'invention contiennent un ou plusieurs excipients ou véhicules tels que des diluants, des lubrifiants, des liants, des agents de désintégration, des colorants, des édulcorants, des arômes.

A titre d'exemple d'excipients ou véhicules, on peut citer :

- ♦ *pour les diluants :* le lactose, le dextrose, le sucrose, le mannitol, le sorbitol, la cellulose,
- ♦ *pour les lubrifiants :* la silice, le talc, l'acide stéarique et ses sels de magnésium et de calcium, le polyéthylène glycol,
- ♦ *pour les liants :* le silicate d'aluminium et de magnésium, l'amidon, la gélatine, la méthylcellulose, la carboxyméthylcellulose de sodium et la polyvinylpyrrolidone, la maltodextrine,
- ♦ *pour les désintégrants :* l'acide alginique et son sel de sodium, les mélanges effervescents, la carboxyméthylcellulose, la croscarmellose sodique,
- ♦ *pour les édulcorants :* l'aspartame, l'acésulfame, le sucralose.

Le pourcentage de sel de strontium dans la composition pharmaceutique est préférentiellement compris entre 40% et 99.9% en poids.

La quantité de sel de strontium dans la composition pharmaceutique est préférentiellement comprise entre 200 mg et 2 g.

La quantité de vitamine $D_3$ dans la composition pharmaceutique est préférentiellement comprise entre 5 μg (200 UI) et 175 μg (7000 UI).

La quantité de cyclodextrine dans la composition pharmaceutique est préférentiellement comprise entre 200 μg et 140 mg, plus préférentiellement entre 2 mg et 70 mg.

Le rapport massique entre la quantité de vitamine D et la quantité de cyclodextrine est préférentiellement compris entre 1/40 et 1/800.

La présente invention concerne également l'utilisation des compositions pharmaceutiques selon l'invention dans le traitement des maladies osseuses, plus particulièrement l'ostéopénie et l'ostéoporose, et dans le traitement de l'arthrose.

## ABREVIATIONS / SIGLES

| DIMEB | heptakis(2,6-di-*O*-méthyl)-β-cyclodextrine. Le degré de substitution du DIMEB est de 14 groupements méthyle / cyclodextrine. |
|---|---|
| HPBCD | hydroxypropyl-(β-cyclodextrine. |
| HR | humidité relative |
| RAMEB | (β-cyclodextrine statistiquement méthylée (*RA*ndomly *ME*thylated Betacyclodextrin). Le degré de substitution moyen du RAMEB est de 12.6 groupements méthyle / cyclodextrine. |
| SBECD | sulfobutyléther-β-cyclodextrine |
| UI | unités internationales. 1000UI = 25 $\mu$g vitamine D. |
| TRIMEB | heptakis (2,3,6-tri-*O*-méthyl)-β-cyclodextrine. Le degré de substitution du TRIMEB est de 21 groupements méthyle / cyclodextrine. |

**Les exemples ci-dessous illustrent l'invention.**

## EXEMPLE 1 : Complexe de vitamine $D_3$ et de RAMEB :

### Exemple 1 A

25 $\mu$g de cholécalciférol sont mélangés à 0.975 mg de RAMEB dans de l'eau ou du tert-butanol, puis le solvant est éliminé par atomisation ou lyophilisation.

### Exemple 1 B

25 $\mu$g de cholécalciférol sont mélangés à 9.975 mg de RAMEB dans de l'eau ou du tert-butanol, puis le solvant est éliminé par atomisation ou lyophilisation.

### Exemple 1 C

25 $\mu$g de cholécalciférol sont mélangés à 19.975 mg de RAMEB dans de l'eau ou du tert-butanol, puis le solvant est éliminé par atomisation ou lyophilisation.

## EXEMPLE 2 : Composition pharmaceutique pour sachet contenant 2 g de ranélate de strontium et 1000 UI de vitamine $D_3$

### Exemple 2A

Le complexe de vitamine $D_3$ et de RAMEB de l'Exemple 1 A est mélangé à 4 g de granules de Protelos® contenant 2 g de ranélate de strontium anhydre.

| | |
|---|---|
| Ranélate de strontium anhydre | 2 g |
| Cholécalciférol | 25 $\mu$g |
| RAMEB | 0.975 mg |
| Aspartame | 20 mg |
| Maltodextrine | 400 mg |
| Mannitol | 948 mg |

Exemple 2B

Le complexe de vitamine D$_3$ et de RAMEB de l'Exemple 1 B est mélangé à 4 g de granules de Protelos® contenant 2 g de ranélate de strontium anhydre.

| | |
|---|---|
| Ranélate de strontium anhydre | 2 g |
| Cholécalciférol | 25 µg |
| RAMEB | 9.975 mg |
| Aspartame | 20 mg |
| Maltodextrine | 400 mg |
| Mannitol | 948 mg |

Exemple 2C

Le complexe de vitamine D$_3$ et de RAMEB de l'Exemple 1C est mélangé à 4 g de granules de Protelos® contenant 2 g de ranélate de strontium anhydre.

| | |
|---|---|
| Ranélate de strontium anhydre | 2 g |
| Cholécalciférol | 25 µg |
| RAMEB | 19.975 mg |
| Aspartame | 20 mg |
| Maltodextrine | 400 mg |
| Mannitol | 948 mg |

**EXEMPLE 3 : Comprimé contenant 600 mg de malonate de strontium et 500 UI de vitamine D$_3$**

Exemple 3A

| | |
|---|---|
| Malonate de strontium anhydre | 600 mg |
| Cholécalciférol | 12.5 µg |
| RAMEB | 487.5 µg |
| Cellulose microcristalline | 87 mg |
| Polyvidone | 24 mg |
| Silice colloïdale anhydre | 5 mg |
| Stéarate de magnésium | 5 mg |

Exemple 3B

| | |
|---|---|
| Malonate de strontium anhydre | 600 mg |
| Cholécalciférol | 12.5 µg |
| RAMEB | 9.9875 mg |
| Cellulose microcristalline | 87 mg |
| Polyvidone | 24 mg |
| Silice colloïdale anhydre | 5 mg |
| Stéarate de magnésium | 5 mg |

Préparation du comprimé de l'Exemple 3.

*Pour environ 5000 comprimés :*

3000 g de malonate de strontium et 170 g de cellulose microcristalline sont soigneusement mélangés. Le mélange est tamisé, puis 120 g de Polyvidone et de l'eau purifiée (qsp obtenir un granulat homogène -environ 375 g) sont ajoutés. Le granulat est tamisé, séché à 40°C pendant 2h1/2 à 3h, puis tamisé à nouveau.
25 g de silice colloïdale anhydre et 265 g de cellulose microcristalline sont soigneusement mélangés et tamisés, puis ajoutés au granulat préparé précédemment et au complexe de l'Exemple 1 (2.5 g du complexe 1 A lorsqu'on veut préparer des comprimés selon l'Exemple 3A; 50 g du complexe 1 C lorsqu'on veut préparer des comprimés selon l'Exemple 3B). 300 g de ce mélange sont ajoutés à 25 g de stéarate de magnésium tamisé, puis, lorsqu'un mélange homogène est obtenu, le reste du mélange est ajouté.
Le mélange final est comprimé.

## EXEMPLE 4 : Comprimé contenant 798 mg d'acétate de strontium et 500 UI de vitamine $D_3$

Exemple 4A

| | |
|---|---|
| Acétate de strontium anhydre | 798 mg |
| Cholécalciférol | 12.5 $\mu$g |
| RAMEB | 487.5 $\mu$g |
| Cellulose microcristalline | 116 mg |
| Polyvidone | 32 mg |
| Silice colloïdale anhydre | 6.66 mg |
| Stéarate de magnésium | 6.66 mg |

Exemple 4B

| | |
|---|---|
| Acétate de strontium anhydre | 798 mg |
| Cholécalciférol | 12.5 $\mu$g |
| RAMEB | 9.9875 mg |
| Cellulose microcristalline | 116 mg |
| Polyvidone | 32 mg |
| Silice colloïdale anhydre | 6.66 mg |
| Stéarate de magnésium | 6.66 mg |

Préparation du comprimé de l'Exemple 4.

*Pour environ 5000 comprimés :*

3990 g d'acétate de strontium et 227 g de cellulose microcristalline sont soigneusement mélangés. Le mélange est tamisé, puis 160 g de Polyvidone et de l'eau purifiée (qsp obtenir un granulat homogène -environ 500 g) sont ajoutés. Le granulat est tamisé, séché à 40°C pendant 2h1/2 à 3h, puis tamisé à nouveau. 33.3 g de silice colloïdale anhydre et 353 g de cellulose microcristalline sont soigneusement mélangés et tamisés, puis ajoutés au granulat préparé précédemment et au complexe de l'Exemple 1 (2.5 g du complexe 1A lorsqu'on veut préparer des comprimés selon l'Exemple 4A; 50 g du complexe 1 C lorsqu'on veut préparer des comprimés selon l'Exemple 4B).
400 g de ce mélange sont ajoutés à 33.3 g de stéarate de magnésium tamisé, puis, lorsqu'un mélange homogène est obtenu, le reste du mélange est ajouté.
Le mélange final est comprimé.

EXEMPLE 5 : Comprimé contenant 790 mg de succinate de strontium et 500 UI de vitamine D₃

Exemple 5A

| | |
|---|---|
| Succinate de strontium anhydre | 790 mg |
| Cholécalciférol | 12.5 µg |
| RAMEB | 487.5 µg |
| Cellulose microcristalline | 114.5 mg |
| Polyvidone | 31.6 mg |
| Silice colloïdale anhydre | 6.6 mg |
| Stéarate de magnésium | 6.6 mg |

Exemple 5B

| | |
|---|---|
| Succinate de strontium anhydre | 790 mg |
| Cholécalciférol | 12.5 µg |
| RAMEB | 9.9875 mg |
| Cellulose microcristalline | 114.5 mg |
| Polyvidone | 31.6 mg |
| Silice colloïdale anhydre | 6.6 mg |
| Stéarate de magnésium | 6.6 mg |

Préparation du comprimé de l'Exemple 5.

*Pour environ 5000 comprimés :*

3950 g de succinate de strontium et 224 g de cellulose microcristalline sont soigneusement mélangés. Le mélange est tamisé, puis 158 g de Polyvidone et de l'eau purifiée (qsp obtenir un granulat homogène -environ 500 g) sont ajoutés. Le granulat est tamisé, séché à 40°C pendant 2hl/2 à 3h, puis tamisé à nouveau.
33 g de silice colloïdale anhydre et 348 g de cellulose microcristalline sont soigneusement mélangés et tamisés, puis ajoutés au granulat préparé précédemment et au complexe de l'Exemple 1 (2.5 g du complexe 1A lorsqu'on veut préparer des comprimés selon l'Exemple 5A; 50 g du complexe 1C lorsqu'on veut préparer des comprimés selon l'Exemple 5B). 400 g de ce mélange sont ajoutés à 33 g de stéarate de magnésium tamisé, puis, lorsqu'un mélange homogène est obtenu, le reste du mélange est ajouté.
Le mélange final est comprimé.

EXEMPLE 6 : Comprimé contenant 900 mg de cétoglutarate de strontium et 500 UI de vitamine D₃

Exemple 6A

| | |
|---|---|
| Cétoglutarate de strontium anhydre | 900 mg |
| Cholécalciférol | 12.5 µg |
| RAMEB | 487.5 µg |
| Cellulose microcristalline | 130.5 mg |
| Polyvidone | 36 mg |
| Silice colloïdale anhydre | 7.5 mg |
| Stéarate de magnésium | 7.5 mg |

Exemple 6B

| | |
|---|---|
| Cétoglutarate de strontium anhydre | 900 mg |
| Cholécalciférol | 12.5 μg |
| RAMEB | 9.9875 mg |
| Cellulose microcristalline | 130.5 mg |
| Polyvidone | 36 mg |
| Silice colloïdale anhydre | 7.5 mg |
| Stéarate de magnésium | 7.5 mg |

Préparation du comprimé de l'Exemple 6.

*Pour environ 5000 comprimés :*

4500 g de cétoglutarate de strontium et 255 g de cellulose microcristalline sont soigneusement mélangés. Le mélange est tamisé, puis 180 g de Polyvidone et de l'eau purifiée (qsp obtenir un granulat homogène -environ 560 g) sont ajoutés. Le granulat est tamisé, séché à 40°C pendant 2h1/2 à 3h, puis tamisé à nouveau. 37.5 g de silice colloïdale anhydre et 397 g de cellulose microcristalline sont soigneusement mélangés et tamisés, puis ajoutés au granulat préparé précédemment et au complexe de l'Exemple 1 (2.5 g du complexe 1 A lorsqu'on veut préparer des comprimés selon l'Exemple 6A; 50 g du complexe 1C lorsqu'on veut préparer des comprimés selon l'Exemple 6B).
525 g de ce mélange sont ajoutés à 37.5 g de stéarate de magnésium tamisé, puis, lorsqu'un mélange homogène est obtenu, le reste du mélange est ajouté.
Le mélange final est comprimé.

## EXEMPLE 7 : Stabilité du complexe vitamine D$_3$+RAMEB de l'Exemple 1B

La stabilité à 40°C/75% HR du complexe vitamine D$_3$+RAMEB de l'Exemple 1B a été testée et comparée à la stabilité de : 1) la vitamine D$_3$ pure, 2) un concentrat de vitamine D$_3$ sous forme poudre (DSM).
L'étude est réalisée dans des piluliers en verre brun étanche (bouchon type antibiotique chlorobutyl gris D 13 - cap aluminium naturel à sertir D20mm, opercule déchirable).

| | % vitamine D$_3$ | | |
|---|---|---|---|
| t | vitamine D3 pure | concentrat de vitamine D$_3$ | **complexe vitamine D$_3$+RAMEB (Exemple 1B)** |
| t0 | 98.0 | 92.7 | **94.3** |
| t0 + 3 semaines 40°C/75% HR | 20.3 | 87.4 | **93.6** |
| t0 + 6 semaines 40°C/75% HR | 23.1 | 88.1 | **94.3** |

Le tableau ci-dessus montre que le complexe de vitamine D$_3$ et de RAMEB selon l'invention a une stabilité améliorée.

EXEMPLE 8 : Stabilité de la composition pharmaceutique de l'Exemple 2B.

La stabilité de la composition pharmaceutique de l'Exemple 2B selon l'invention en sachet a été testée dans différentes conditions de température et d'humidité.
Les sachets sont constitués d'un complexe multicouche (papier/polyethylène/aluminium/polyethylène).

| Sachet | | | |
|---|---|---|---|
| t | teneur en vitamine $D_3$ (UI) | | |
| | 25°C/60% HR | 30°C/65% HR | 40°C/75% HR |
| t0 | 1011.8 | | |
| t0 + 6 semaines | 997.6 | 999.3 | 1014.9 |
| t0 + 3 mois | 983.6 | 1000.0 | 986.1 |
| t0 + 6 mois | 1017.9 | 998.2 | 998.0 |

Le tableau ci-dessus montre que la vitamine D contenue dans la formulation sachet de ranélate de strontium, de vitamine D et de cyclodextrine selon l'invention a une excellente stabilité, même dans des conditions de température et d'humidité élevées (40°C/75% HR).

**EXEMPLE 9 : Uniformité de teneur (vitamine $D_3$) de la composition pharmaceutique de l'Exemple 2B.**

L'essai est effectué sur 10 sachets.
Le contenu de chaque sachet est introduit dans un flacon conique puis 25 ml de méthanol sont ajoutés. Le mélange est agité 1h, puis centrifugé 10 mn à 4000 rotations par minute. Une solution de référence de vitamine $D_3$ dans le méthanol (concentration $1 \mu g/ml$) est également préparée.
Les solutions à tester sont dosées en utilisant la technique de chromatographie liquide phase inverse, avec une détection par spectrophotométrie UV.
La teneur en vitamine $D_3$ $X_i$ du $i^è$ sachet (i allant de 1 à 10) est calculée de la façon suivante :

$$X_i = AT_i / AR$$

où $AT_i$ est l'aire sous le pic de vitamine $D_3$ pour le $i^è$ sachet,
et AR est l'aire sous le pic de vitamine $D_3$ dans le chromatogramme de la solution de référence,

La teneur moyenne $X_m$ est exprimée de la façon suivante :

$$X_m = (\Sigma X_i)/10$$

La valeur d'acceptation (AV), exprimée en pourcentage de la valeur théorique, est donné par la formule suivante :

$$AV = (M - X_m) + k \text{ x } s$$

où :

$X_m$ est la teneur moyenne, exprimée en pourcentage de la valeur théorique;
M est la valeur de référence, exprimée en pourcentage de la valeur théorique : M=98.5 si
$X_m < 98.5$; $M = X_m$ si $98.5 \leq X_m \leq 101.5$; M=101.5 si $X_m > 101.5$;
k est la constante d'acceptabilité (k=2.4 pour 10 sachets);
s est l'écart type des teneurs $X_i$.

Résultats :

| Paramètres d'uniformité de teneur (vitamine $D_3$) | Lot L0027602 (sachet) selon l'Exemple 2B |
|---|---|
| Teneur moyenne | 94.4% |
| Coefficient de variation | 2.3% |
| Valeur d'acceptation (AV) | 9.4 |

Selon la Pharmacopée Européenne, article 2.9.40, une valeur d'acceptation inférieure à 15 signifie que l'uniformité de teneur est conforme (niveau L1).

Le tableau ci-dessus montre donc que la vitamine D contenue dans la formulation sachet de ranélate de strontium, de vitamine D et de cyclodextrine selon l'invention a une uniformité de teneur qui répond aux exigences réglementaires.

## Revendications

1. Composition pharmaceutique comprenant comme principes actifs un sel de strontium et de la vitamine D, et comme excipients une cyclodextrine, ainsi que un ou plusieurs autres excipients ou véhicules inertes, non toxiques, pharmaceutiquement acceptables.

2. Composition pharmaceutique selon la revendication 1, dans laquelle la vitamine D est le cholécalciférol (vitamine $D_3$).

3. Composition pharmaceutique selon la revendication 2, dans laquelle la vitamine $D_3$ est dosée à 1000 UI.

4. Composition pharmaceutique selon l'une quelconque des revendications 1 à 3, dans laquelle la cyclodextrine est une (β-cyclodextrine substituée.

5. Composition pharmaceutique selon la revendication 4, dans laquelle la β-cyclodextrine est substituée par un ou plusieurs groupements méthyle, hydroxypropyle ou sulfobutyléther.

6. Composition pharmaceutique selon la revendication 5, dans laquelle la β-cyclodextrine substituée est choisie parmi les HPBCD (hydroxypropyl-β-cyclodextrines), les SBECD (sulfobutyléther-β-cyclodextrines) et les β-cyclodextrines méthylées ou partiellement méthylées.

7. Composition pharmaceutique selon la revendication 6, dans laquelle la β-cyclodextrine substituée est le RAMEB.

8. Composition pharmaceutique selon l'une quelconque des revendications 1 à 7, dans laquelle le rapport massique entre la quantité de vitamine D et la quantité de cyclodextrine est compris entre 1/40 et 1/800.

9. Composition pharmaceutique selon l'une quelconque des revendications 1 à 8, dans laquelle le sel de strontium est choisi parmi le ranélate de strontium, le malonate de strontium, l'acétate de strontium, le L-ascorbate de strontium, l'aspartate de strontium, le borate de strontium, le camphorate de strontium, le carbonate de strontium, le cétoglutarate de strontium, le citrate de strontium, l'éthanesulfonate de strontium, le formate de strontium, le fumarate de strontium, le gluconate de strontium, le glutamate de strontium, l'hydrogénophosphate de strontium, le lactate de strontium, le L-lactate de strontium, le L-malate de strontium, le maléate de strontium, le méthanesulfonate de strontium, le nitrate de strontium, l'oxalate de strontium, le phosphate de strontium, le propanesulfonate de strontium, le succinate de strontium, le sulfate de strontium, le tartrate de strontium et leurs hydrates.

10. Composition pharmaceutique selon l'une quelconque des revendications 1 à 9, sous la forme de comprimés à avaler, de comprimés à croquer, de comprimés effervescents, de comprimés dispersibles ou de granules pour sachet.

11. Composition pharmaceutique selon la revendication 9, dans laquelle le sel de strontium est le ranélate de strontium.

12. Composition pharmaceutique selon la revendication 11 sous la forme de granules pour sachet.

13. Composition pharmaceutique selon l'une quelconque des revendications 1 à 12, pour son utilisation dans le traitement des maladies osseuses ou de l'arthrose.

Europäisches
Patentamt

European
Patent Office

Office européen
des brevets

**RAPPORT DE RECHERCHE EUROPEENNE**

Numéro de la demande

EP 10 29 0628

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y,D | CN 1 823 764 A (CHONGQING MEDICINE INDUSTRY IN [CN]) 30 août 2006 (2006-08-30) * revendications 1, 5, 6; exemple 2 * & CHONGQING MEDICINE INDUSTRY IN [CN], machine translation: , 30 août 2006 (2006-08-30), Extrait de l'Internet: URL:http://c2e.cnpat.com.cn/Translationtoen.aspx?AppNo=EACBGCECECCBKAEIDBBHDBCFFAFBKCDFHBCIFCCDIACCEBAA&Doc=XAAAaAFI&FileType=ZCEC [extrait le 2010-03-15] * http://c2e.cnpat.com.cn/Translationtoen.aspx?AppNo=EACBGCECECCBKAEIDBBHDBCFFAFBKCDFHBCIFCCDIACCEBAA&Doc=XAAAaAFI&FileType=ZCEC * ----- | 1-13 | INV. A61K31/593 A61K33/24 A61K47/40 A61K45/06 A61P19/00 |
| Y | DE 10 2005 017775 A1 (SCHERING AG [DE]) 19 octobre 2006 (2006-10-19) * alinéas [0037], [0 39], [0 42] * ----- | 1-13 | **DOMAINES TECHNIQUES RECHERCHES (IPC)** A61K |
| Y | WO 2005/123130 A2 (OSTEOLOGIX AS [DK]; CHRISTGAU STEPHAN [DK]; HANSEN CHRISTIAN [DK]; NIL) 29 décembre 2005 (2005-12-29) * revendications 1, 5, 6, 8 * ----- | 9,11 | |
| Y | US 2007/218111 A1 (EHRENKRANZ JOEL [US] ET AL) 20 septembre 2007 (2007-09-20) * abrégé * * alinéa [0003] * * alinéa [0028] * * revendications 1, 8 * ----- | 1-13 | |

| Le présent rapport a été établi pour toutes les revendications | | |
|---|---|---|
| Lieu de la recherche Munich | Date d'achèvement de la recherche 4 février 2011 | Examinateur Uryga-Polowy, V |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
........................................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 10 29 0628

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

04-02-2011

| Document brevet cité au rapport de recherche | Date de publication | Membre(s) de la famille de brevet(s) | Date de publication |
|---|---|---|---|
| CN 1823764 A | 30-08-2006 | AUCUN | |
| DE 102005017775 A1 | 19-10-2006 | AUCUN | |
| WO 2005123130 A2 | 29-12-2005 | AUCUN | |
| US 2007218111 A1 | 20-09-2007 | AUCUN | |

EPO FORM P0460

Pour tout renseignement concernant cette annexe : voir Journal Officiel de l'Office européen des brevets, No.12/82

## RÉFÉRENCES CITÉES DANS LA DESCRIPTION

**Documents brevets cités dans la description**

- EP 0415850 A
- EP 0813869 A
- EP 1534305 A
- EP 1845082 A
- WO 2004098618 A
- CN 1823764